(19) Europäisches Patentamt
European Patent Office
Office européen des brevets

(11) Veröffentlichungsnummer: **0 262 395 A2**

(12) # EUROPÄISCHE PATENTANMELDUNG

(21) Anmeldenummer: **87112383.2**

(22) Anmeldetag: **26.08.87**

(51) Int. Cl.[4]: **C07D 207/335**, C07D 307/52, C07D 333/20, C07D 409/04, C07D 405/04, A61K 31/36, A61K 31/40, A61K 31/34, A61K 31/50

(30) Priorität: **03.09.86 DE 3629929**

(43) Veröffentlichungstag der Anmeldung: **06.04.88 Patentblatt 88/14**

(84) Benannte Vertragsstaaten: **AT BE CH DE ES FR GB GR IT LI LU NL SE**

(71) Anmelder: **Dr. Karl Thomae GmbH**
**Postfach 1755**
**D-7950 Biberach (Riss)(DE)**

(72) Erfinder: **Heckel, Armin, Dr.Dipl.-Chem.**
**Lamparterweg 1**
**D-7950 Biberach 1(DE)**
Erfinder: **Nickl. Josef, Dr. Dipl.-Chem.**
**Silcherstrasse 8**
**D-7950 Biberach 1(DE)**
Erfinder: **Müller, Erich, Dr. Dipl.-Chem.**
**Talfeldstrasse 34**
**D-7950 Biberach 1(DE)**
Erfinder: **Narr, Berthold, Dr. Dipl.-Chem.**
**Obere Au 5**
**D-7950 Biberach 1(DE)**
Erfinder: **Weisenberger, Johannes. Dr. Dipl.-Chem.**
**Haydnweg 5**
**D-7950 Biberach 1(DE)**
Erfinder: **Eisert, Wolfgang, Prof. Dr. Dr. Dr.**
**Friedrich-Goll-Weg 5**
**D-7950 Biberach 1(DE)**
Erfinder: **Müller, Thomas, Dr. Dipl.-Chem.**
**Gymnasiumstrasse 16**
**D-7950 Biberach 1(DE)**

(54) **Neue Sulfonamido-äthylverbindungen, diese Verbindungen enthaltende Arzneimittel und Verfahren zu ihrer Herstellung.**

(57) Die Erfindung betrifft neue Sulfonamido-äthylverbindungen der allgemeinen Formel

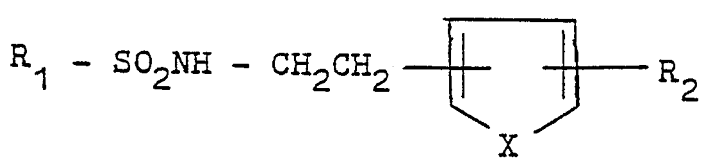

(I)

EP 0 262 395 A2

in der

$R_1$ eine gegebenenfalls durch ein Halogenatom, eine Methyl-oder Methoxygruppe mono-, di-oder trisubstituierte Phenylgruppe, wobei die Substituenten gleich oder verschieden sein können, eine Benzyl-, Nitrophenyl-, Acetamidophenyl-oder Thienylgruppe,

$R_2$ eine über eine geradkettige oder verzweigte Alkylengruppe oder über eine Alkenylengruppe gebundene Hydroxycarbonyl-oder Alkoxycarbonylgruppe, wobei jeweils eine Methylengruppe der vorstehend erwähnten Alkylen-oder Alkenylenreste, welche mit dem heterocyclischen Rest verknüpft sein muß, durch eine Hydroxymethylen-oder Carbonylgruppe ersetzt sein kann, oder eine gegebenenfalls im Kohlenstoffgerüst durch eine Alkylgruppe substituierte 4,5-Dihydro-pyridazin-3-on-6-yl-oder Pyridazin-3-on-6-yl-gruppe und

X eine gegebenenfalls durch eine Alkylgruppe substituierte Iminogruppe, ein Sauerstcff-oder Schwefelatom bedeuten, deren Enantiomere und deren Salze mit anorganischen oder organischen Basen, falls $R_2$ eine Hydroxycarbonylgruppe enthält.

Die neuen Verbindungen wertvolle pharmakologische Eigenschaften auf, insbesondere antithrombotische Wirkungen, außerdem stellen diese Thromboxanantagonisten dar, und lassen sich nach an und für sich bekannten Verfahren herstellen.

# Neue Sulfonamido-äthylverbindungen, diese Verbindungen enthaltende Arzneimittel und Verfahren zu ihrer Herstellung

Gegenstand der vorliegenden Erfindung sind neue Sulfonamido-äthylverbindungen der allgemeinen Formel

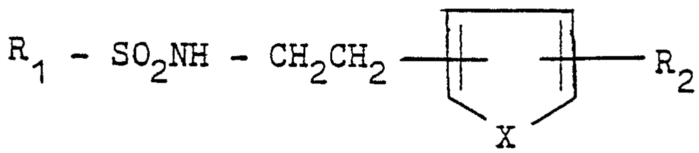

(I)

deren Enantiomere und deren Additionssalze, insbesondere für die pharmazeutische Anwendung deren physiologisch verträgliche Additionssalze mit anorganischen oder organischen Basen, falls $R_2$ eine Hydroxycarbonylgruppe enthält, welche wertvolle pharmakologische Eigenschaften aufweisen, insbesondere antithrombotische Wirkungen, außerdem stellen die neuen Verbindungen Thromboxanantagonisten dar.

Gegenstand der vorliegenden Erfindung sind somit die neuen Verbindungen der obigen allgemeinen Formel I, deren Enantiomere, falls der Rest $R_2$ ein optisch aktives Kohlenstoffatom enthält, deren Additionssalze mit anorganischen oder organischen Basen, insbesondere für die pharmazeutische Verwendung deren physiologisch verträgliche Additionssalze, diese Verbindungen enthaltende Arzneimittel und Verfahren zu ihrer Herstellung.

In der obigen allgemeinen Formel bedeutet

$R_1$ eine gegebenenfalls durch ein Halogenatom, eine Methyl-oder Methoxygruppe mono-, di-oder trisubstituierte Phenylgruppe, wobei die Substituenten gleich oder verschieden sein können, eine Benzyl-, Nitrophenyl-, Acetamidophenyl-oder Thienylgruppe,

$R_2$ eine über eine geradkettige oder verzweigte Alkylengruppe mit 1 bis 5 Kohlenstoffatomen oder über eine Alkenylengruppe mit 2 bis 5 Kohlenstoffatomen gebundene Hydroxycarbonyl-oder Alkoxycarbonylgruppe mit insgesamt 2 bis 4 Kohlenstoffatomen, wobei jeweils eine Methylengruppe der vorstehend erwähnten Alkylen-oder Alkenylenreste, welche mit dem heterocyclischen Rest verknüpft sein muß, durch eine Hydroxymethylen-oder Carbonylgruppe ersetzt sein kann, oder eine gegebenenfalls im Kohlenstoffgerüst durch eine Alkylgruppe mit 1 bis 3 Kohlenstoffatomen substituierte 4,5-Dihydro-pyridazin-3-on-6-yl-oder Pyridazin-3-on-6-yl-gruppe und

X eine gegebenenfalls durch eine Alkylgruppe mit 1 bis 3 Kohlenstoffatomen substituierte Iminogruppe, ein Sauerstoff-oder Schwefelatom.

Für die bei der Definition der Reste X, $R_1$ und $R_2$ eingangs erwähnten Bedeutungen kommt beispielsweise

für $R_1$ die der Benzyl-, Phenyl-, 2-Methylphenyl-, 3-Methylphenyl-, 4-Methylphenyl-, 2-Methoxyphenyl-, 3-Methoxyphenyl-, 4-Methoxyphenyl-, 2-Fluorphenyl-, 3-Fluorphenyl-, 4-Fluorphenyl-, 2-Chlorphenyl-, 3-Chlorphenyl-, 4-Chlorphenyl-, 2-Bromphenyl-, 4-Bromphenyl-, 3,4-Dimethylphenyl-, 3,4-Dimethoxyphenyl-, 2,4-Difluorphenyl-, 2,4-Dichlorphenyl-, 2,5-Dichlorphenyl-, 2,4-Dibromphenyl-, 4-Methyl-2-chlorphenyl-, 2,4,5-Trichlorphenyl-, 4-Nitrophenyl-, 4-Acetamidophenyl-oder Thien-2-ylgruppe,

für $R_2$ die der Hydroxycarbonylmethyl-, 1-Hydroxycarbonyläthyl-, 2-Hydroxycarbonyl-äthyl-, 1-Hydroxycarbonyl-propyl-, 3-Hydroxycarbonyl-propyl-, 1-Hydroxycarbonyl-butyl-, 4-Hydroxycarbonyl-butyl-, 1-Hydroxycarbonyl-1-methyl-äthyl-, 2-Hydroxycarbonyl-1-methyl-äthyl-, 2-Hydroxycarbonyl-äthenyl-, 2-Hydroxycarbonyl-1-methyl-äthenyl-, 3-Hydroxycarbonyl-propenyl-, 2-Hydroxycarbonyl-äthanon-(1)-yl-, 3-Hydroxycarbonyl-n-propanon-(1)-yl-, 4-Hydroxycarbonyl-n-butanon-(1)-yl-, 5-Hydroxycarbonyl-n-pentanon-(1)-yl-, 2-Hydroxycarbonyl-2-methyl-äthanon-(1)-yl-, 3-Hydroxycarbonyl-2-methyl-n-propanon-(1)-yl-, 3-Hydroxycarbonyl-3-methyl-n-propanon-(1)-yl-, 4-Hydroxycarbonyl-2-methyl-n-butanon-(1)-yl-, 4-Hydroxycarbonyl-3-methyl-n-butanon-(1)-yl-, 4-Hydroxycarbon-yl-4-methyl-n-butanon-(1)-yl-, 2-Hydroxycarbonyl-2-äthyläthanon-(1)-yl-, 2-Hydroxycarbonyl-2-n-propyläthanon-(1)-yl-, 2-Hydroxycarbonyl-2-äthyl-n-propanon-(1)-yl-, 3-Hydroxycarbonyl-3-äthyl-n-propanon-(1)-yl-, 4-Hydroxycarbonyl-n-buten-2-on-(1)-yl-, 5-Hydroxycarbonyl-n-penten-2-on-(1)-yl-, Methoxycarbonylmethyl-, Äthoxycarbonylmethyl-,

Isopropoxycarbonylmethyl-, 2-Methoxycarbonyl-äthyl-, 2-Äthoxycarbonyl-äthyl-, 3-Methoxycarbonyl-propyl-, 4-Äthoxycarbonylbutyl-, 2-Methoxycarbonyl-1-methyl-äthyl-, 2-Äthoxycarbonyl-1-methyl-äthyl-, 2-Isopropoxycarbonyl-1-methyl-äthyl-, 1-Methoxycarbonyl-äthyl-, 1-Äthoxycarbonyl-äthyl-, 1-Methoxycarbonyl-propyl-, 1-Äthoxycarbonyl-butyl-, 1-Methoxycarbonyl-1-methyl-äthyl-, 1-Äthoxycarbonyl-1-methyl-äthyl-, 1-Isopropoxycarbonyl-1-methyl-äthyl-, 2-Methoxycarbonyläthenyl-, 2-Methoxycarbonyl-1-methyläthenyl-, 2-Äthoxycarbonyl-1-methyl-äthenyl-, 3-Methoxycarbonylpropenyl-, 2-Methoxycarbonyl-äthanon-(1)-yl-, 3-Methoxycarbonyl-n-propanon-(1)-yl-, 3-Äthoxycarbonyl-n-propanon-(1)-yl-, 3-n-Propoxycarbonyl-n-propanon-(1)-yl-, 4-Äthoxycarbonyl-n-butanon-(1)-yl-, 5-Äthoxycarbon-yl-n-pentanon-(1)-yl-, 2-Äthoxycarbonyl-2-methyl-äthanon-(1)-yl-, 3-Äthoxycarbonyl-2-methyl-n-propanon-(1)-yl-, 3-Äthoxycarbonyl-3-methyl-n-propanon-(1)-yl-, 4-Äthoxycarbonyl-2-methyl-n-butanon-(1)-yl-, 4-Äthoxycarbonyl-3-methyl-n-butanon-(1)-yl-, 4-Äthoxycarbonyl-4-methyl-n-butanon-(1)-yl-, 2-Äthoxycarbonyl-2-äthyl-äthanon-(1)-yl-, 2-Äthoxycarbonyl-2-n-propyl-äthanon-(1)-yl-, 3-Äthoxycarbonyl-2-äthyl-n-propanon-(1)-yl-, 3-Äthoxycarbonyl-3-äthyl-n-propanon-(1)-yl-, 3-Äthoxycarbonyl-n-propanon-(1)-yl-, 3-Äthoxycarbonyl-2-methyl-n-propanon-(1)-yl-, 3-Äthoxycarbonyl-3-methyl-n-propanon-(1)-yl-, 4-Äthoxycarbonyl-n-buten-2-on-(1)-yl-, 5-Äthoxycarbonyl-n-penten-2-on-(1)-yl-, 4,5-Dihydro-pyridazin-3-on-6-yl-, 4,5-Dihydro-5-methyl-pyridazin-3-on-6-yl-, 4,5-Dihydro-5-äthyl-pyridazin-3-on-6-yl-, 4,5-Dihydro-5-n-propyl-pyridazin-3-on-6-yl-, Pyridazin-3-on-6-yl-, 5-Methyl-pyridazin-3-on-6-yl-, 5-Äthylpyridazin-3-on-6-yl-, 5-n-Propyl-pyridazin-3-on-6-yl-oder 5-Isopropyl-pyridazin-3-on-6-yl-gruppe und

für X die des Sauerstoff-oder Schwefelatoms der Imino-, Methylimino-, Ethylimino-oder Isopropyliminogruppe in Betracht.

Beispielsweise seien folgende Verbindungen genannt, die unter die vorstehende allgemeine Formel I fallen:

4-[2-(2-(p-Chlorbenzolsulfonylamino)-äthyl)-1-methylpyrrol-5-yl]-4-oxobuttersäure-methylester

4-[2-(2-Benzolsulfonylaminoethyl)-1-methylpyrrol-5-yl]-4-oxobuttersäure-methylester

4-[2-(2-(p-Fluorbenzolsulfonylamino)-äthyl)-1-methylpyrrol-5-yl]-4-oxobuttersäure-methylester

4-[2-(2-Benzolsulfonylamino-äthyl)-1-methylpyrrol-5-yl]-4-oxobuttersäure-methylester

4-[2-(2-Thiophen-2-ylsulfonylamino-äthyl)-1-methylpyrrol-5-yl]-4-oxobuttersäure-methylester

4-[2-(2,4,5-Trichlorbenzolsulfonylamino)-äthyl)-1-methyl-pyrrol-5-yl]-4-oxobuttersäure-methylester

4-[2-(2-(p-Toluolsulfonylamino)-äthyl)-1-methylpyrrol-5-yl]-4-oxobuttersäure-methylester

4-[2-(2-(p-Acetamidobenzolsulfonylamino)-äthyl)-1-methylpyrrol-5-yl]-4-oxobuttersäure-methylester

4-[2-(2-(p-Nitrobenzolsulfonylamino)-äthyl)-1-methylpyrrol-5-yl]-4-oxobuttersäure-methylester

4-[2-(2-(o-Methoxybenzolsulfonylamino)-äthyl)-1-methylpyrrol-5-yl]-4-oxobuttersäure-methylester

4-[2-(2-(p-Chlorbenzolsulfonylamino)-äthyl)-1-methylpyrrol-5-yl]-4-oxobuttersäure

4-[2-(2-Benzolsulfonylamino-äthyl)-1-methylpyrrol-5-yl]-4-oxobuttersäure

4-[2-(2-(p-Fluorbenzolsulfonylamino)-äthyl)-1-methylpyrrol-5-yl]-4-oxobuttersäure

4-[2-(2-Benzylsulfonylamino-äthyl)-1-methylpyrrol-5-yl]-4-oxobuttersäure

4-[2-(2-Thiophen-2-yl-sulfonylamino-äthyl)-1-methylpyrrol-5-yl]-4-oxobuttersäure

4-[2-(2-(2,4,5-Trichlorbenzolsulfonylamino)-äthyl)-1-methylpyrrol-5-yl]-4-oxobuttersäure

4-[2-(2-(p-Toluolsulfonylamino)-äthyl)-1-methylpyrrol-5-yl]-4-oxo-buttersäure

4-[2-(2-(p-Acetamido-benzolsulfonylamino)-äthyl)-1-methylpyrrol-5-yl]-4-oxobuttersäure

4-[2-(2-(p-Nitrobenzolsulfonylamino)-äthyl)-1-methylpyrrol-5-yl]-4-oxobuttersäure

4-[2-(2-(2,5-Dichlorbenzolsulfonylamino)-äthyl)-1-methylpyrrol-5-yl]-4-oxobuttersäure

4-[2-(2-Benzolsulfonylamino-äthyl)-furan-5-yl]-4-oxobuttersäure

4-[2-(2-Benzolsulfonylamino-äthyl)-thiophen-5-yl]-4-oxobuttersäure

6-[2-(2-Benzolsulfonylamino-äthyl)-1-methylpyrrol-5-yl]-4,5-dihydro-3(2H)pyridazinon

6-[2-(2-(p-Fluorbenzolsulfonylamino)-äthyl)-1-methylpyrrol5-yl]-4,5-dihydro-3(2H)pyridazinon

6-[2-(2-Benzolsulfonylamino-äthyl)-furan-5-yl]-4,5-dihydro3(2H)-pyridazinon

6-[2-(2-Benzolsulfonylamino-äthyl)-thiophen-5-yl]-4,5-dihydro-3(2H)-pyridazinon

4-[2-(2-Benzylsulfonylamino-äthyl)-thiophen-5-yl)-4-oxobuttersäure

[2-(2-(p-Chlorbenzolsulfonylamino)-äthyl)-1-methylpyrrol-5-yl]-essigsäure-methylester

[2-(2-Benzolsulfonylamino-äthyl)-1-methylpyrrol-5-yl]-essigsäure-methylester

[2-(2-(p-Fluorbenzolsulfonylamino)-äthyl)-1-methylpyrrol-5-yl]-essigsäure-methylester

[2-(2-Thiophen-2-ylsulfonylamino-äthyl)-1-methylpyrrol-5-yl]-essigsäure-methylester

[2-(2-(p-Toluolsulfonylamino)-äthyl)-1-methylpyrrol-5-yl]-essigsäure-methylester

[2-(2-(p-Nitrobenzolsulfonylamino)-äthyl)-1-methylpyrrol-5-yl]-essigsäure-methylester

[2-(2-(o-Methoxybenzolsulfonylamino)-äthyl)-1-methylpyrrol-5-yl]-essigsäure-methylester

[2-(2-(p-Chlorbenzolsulfonylamino)-äthyl)-1-methylpyrrol-5-yl]-essigsäure

[2-(2-Benzolsulfonylamino-äthyl)-1-methylpyrrol-5-yl]-essigsäure

[2-(2-(p-Fluorbenzolsulfonylamino)-äthyl)-1-methylpyrrol-5-yl]-essigsäure

[2-(2-Benzylsulfonylamino-äthyl)-1-methylpyrrol-5-yl]-essigsäure

[2-(2-Thiophen-2-yl-sulfonylamino-äthyl)-1-methylpyrrol-5-yl]-essigsäure

[2-(2-(p-Toluolsulfonylamino)-äthyl)-1-methylpyrrol-5-yl]-essigsäure

[2-(2-(p-Nitrobenzolsulfonylamino)-äthyl)-1-methylpyrrol-5-yl]-essigsäure

[2-(2,5-Dichlorbenzolsulfonylamino)-äthyl)-1-methylpyrrol-5-yl]-essigsäure

[2-(2-Benzolsulfonylamino-äthyl)-furan-5-yl]-essigsäure

[2-(2-Benzolsulfonylamino-äthyl)-thiophen-5-yl]-essigsäure

[2-(2-(p-Chlorbenzolsulfonylamino)-äthyl)-thiophen-5-yl]-essigsäure

Bevorzugte Verbindungen der obigen allgemeinen Formel I sind diejenigen, in denen

$R_1$ eine gegebenenfalls durch eine Methyl-, Methoxy-oder Nitrogruppe substituierte Phenylgruppe, eine durch ein Fluor-, Chlor-oder Bromatom mono-, di-oder trisubstituierte Phenylgruppe und

$R_2$ eine Hydroxycarbonylmethyl-, Äthoxycarbonylmethyl-, 2-Äthoxycarbonyl-äthyl-, 3-Hydroxycarbonyl-n-propan-(1)-yl-, 3-Methoxycarbonyl-n-propan-(1)-yl-, 3-Hydroxycarbonyl-n-propan-1-on-(1)-yl-, 3-Methoxycarbonyl-n-propan-1-on-(1)-yl-oder 4,5-Dihydro-pyridazin-3-on-6-yl-gruppe und

X eine Methyliminogruppe, ein Sauerstoff-oder Schwefelatom bedeuten.

Besonders bevorzugte Verbindungen der obigen allgemeinen Formel I sind jedoch diejenigen, in denen

$R_1$ eine gegebenenfalls durch ein Fluor-oder Chloratom, durch eine Methyl-oder Methoxygruppe substituierte Phenylgruppe und

$R_2$ eine Hydroxycarbonylmethyl-, 3-Hydroxycarbonyl-n-propan-1-on-(1)-yl-oder 4,5-Dihydro-pyridazin-3-on-6-yl-gruppe und

X eine Methyliminogruppe, ein Sauerstoff-oder Schwefelatom bedeuten.

Erfindungsgemäß erhält man die neuen Verbindungen nach folgenden Verfahren:

a.) Sulfonylierung einer Verbindung der allgemeinen Formel

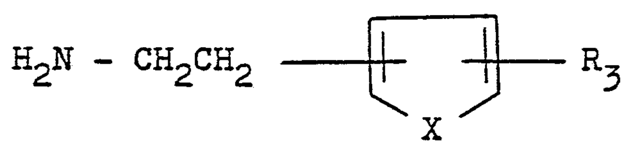

(II)

in der

$R_3$ die für $R_2$ eingangs erwähnten Bedeutungen aufweist, wobei jedoch eine Hydroxygruppe im Rest $R_2$ durch eine hydrolytisch oder hydrogenolytisch abspaltbare Schutzgruppe wie eine Alkoxy-oder Benzyloxygruppe geschützt sein kann, mit einem Phenylsulfonsäurederivat der allgemeinen Formel

$R_1$ -$SO_2Y_1$ ,(III)

in der

$R_1$ wie eingangs definiert ist und

$Y_1$ eine nucleophile Austrittsgruppe wie ein Halogenatom oder eine Alkoxygruppe, z.B. ein Chlor-oder Bromatom, eine Methoxy-oder Äthoxygruppe, darstellt und gegebenenfalls anschließende Abspaltung eines verwendeten Schutzrestes.

Die Umsetzung wird vorzugsweise in einem Lösungsmittel wie Methanol, Äthanol, Wasser/Methanol, Dioxan, Tetrahydrofuran oder Chloroform gegebenenfalls in Gegenwart eines säurebinMittels wie Kaliumcarbonat, Triäthylamin oder Pyridin, wobei die beiden letzteren auch als Lösungsmittel verwendet werden können, zweckmäßigerweise bei Temperaturen zwischen 0 und 50°C, vorzugsweise jedoch bei Raumtemperatur, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators

wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

b.) Zur Herstellung von Verbindungen der allgemeinen Formel I, in der $R_2$ eine Hydroxycarbonylgruppe enthält:

Abspaltung eines Schutzrestes von einer Verbindung der allgemeinen Formel

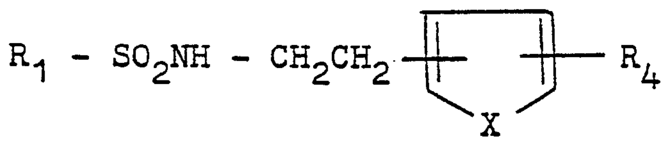

(IV)

in der
$R_1$ wie eingangs definiert ist und
$R_4$ die für $R_2$ eingangs erwähnten Bedeutungen besitzt, wobei jedoch die Carboxygruppe durch einen hydrolytisch, thermolytisch oder hydrogenolytisch abspaltbare Schutzgruppe geschützt ist oder ein funktionelles Derivat der Carboxygruppe darstellt.

Als hydrolysierbare Gruppen kommen beispielsweise funktionelle Derivate der Carboxygruppe wie deren unsubstituierte oder substituierte Amide, Ester, Thioester, Orthoester, Iminoäther, Amidine oder Anhydride, die Nitrilgruppe, Äthergruppen wie die Methoxy-oder Benzyloxygruppe oder Lactone und

als thermolytisch abspaltbare Gruppen beispielsweise Ester mit tertiären Alkoholen, z.B. der tert.Butylester, und als hydrogenolytisch abspaltbare Gruppen beispielsweise Aralkylgruppen, z.B. die Benzylgruppe, in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Enthält beispielsweise eine Verbindung der allgemeinen Formel IV eine Nitril-oder Aminocarbonylgruppe, so können diese Gruppen vorzugsweise mittels 100%iger Phosphorsäure bei Temperaturen zwischen 100 und 180°C, vorzugsweise bei Temperaturen zwischen 120 und 160°C, oder auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C in die Carboxygruppe übergeführt werden.

Enthält beispielsweise eine Verbindung der allgemeinen Formel IV die tert.Butyloxycarbonylgruppe, so kann die tert.Butylgruppe auch thermisch gegebenenfalls in einem inerten Lösungsmittel wie Methlenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 100°C, abgespalten werden.

Enthält beispielsweise eine Verbindung der allgemeinen Formel IV die Benzyloxy-oder Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Methanol/Wasser, Äthanol/Wasser, Eisessig, Essigsäureäthylester, Dioxan oder Dimethylformamid vorzugsweise bei Temperaturen zwischen 0 und 50°C, z.B. bei Raumtemperatur und einem Wasserstoffdruck von 1 bis 5 bar abgespalten werden. Bei der Hydrogenolyse kann gleichzeitig eine halogenhaltige Verbindung enthalogeniert und eine vorhandende Doppelbindung aufhydriert werden.

c.) Zur Herstellung von Verbindungen der allgemeinen Formel I, in der $R_2$ benachbart zum heterocyclischen Rest eine Carbonylgruppe enthält:

Acylierung einer Verbindung der allgemeinen Formel

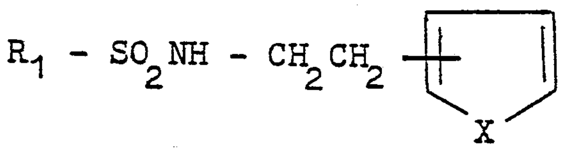
(V)

in der
$R_1$ wie eingangs definiert ist, mit einer Verbindung der allgemeinen Formel

$Y_2$ -$R_5$ ,(VI)

in der
$R_5$ die für $R_2$ eingangs erwähnten Bedeutungen besitzt, wobei jedoch $R_2$ benachbart zu $Y_2$ eine Carbonylgruppe enthalten muß und gleichzeitig eine gegebenenfalls vorhandene Hydroxycarbonylgruppe durch einen hydrolytisch oder hydrogenolytisch abspaltbaren Schutzrest wie eine Alkoxy-oder Benzylgruppe geschützt sein kann, und

$Y_2$ eine nucleophile Austrittsgruppe wie ein Halogenatom, z.B. ein Chlor-, Brom-oder Jodatom, darstellen, oder dessen Anhydrid in Gegenwart einer Lewis-Säure und gegebenenfalls anschließende Abspaltung eines verwendeten Schutzrestes.

Die Friedel-Craft's-Acylierung wird vorzugsweise in einem Lösungsmittel wie Äthylenchlorid oder Nitrobenzol gegebenenfalls in Gegenwart einer Lewis-Säure wie Aluminiumchlorid, Bortrifluorid oder Zinkchlorid zweckmäßigerweise bei Temperaturen zwischen 0 und 50°C, vorzugsweise jedoch bei Raumtemperatur, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperatu ren zwischen 0 und 50°C, vorzugsweise jedoch bei Raumtemperatur, und einem Wasserstoffdruck von 1 bis 7 bar, vorzugsweise jedoch von 3 bis 5 bar.

d.) Zur Herstellung von Verbindungen der allgemeinen Formel I, in der $R_2$ einen Pyridazinonring darstellt:

Umsetzung einer Verbindung der allgemeinen Formel

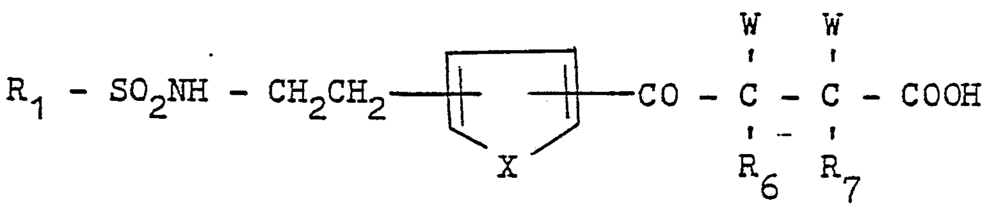
(VII)

in der
$R_1$ wie eingangs definiert ist,
einer der Reste $R_6$ oder $R_7$ ein Wasserstoffatom oder eine Alkylgruppe mit 1 bis 3 Kohlenstoffatomen und der andere der Reste $R_6$ oder $R_7$ ein Wasserstoffatom und

W jeweils ein Wasserstoffatom oder zusammen eine weitere Bindung darstellen, oder deren reaktionsfähige Derivate wie deren Ester, Amide oder Halogenide mit Hydrazin.

Die Umsetzung wird zweckmäßigerweise in einem Lösungsmittel wie Methanol, Äthanol, Isopropanol, Eisessig, Propionsäure und/oder in einem Überschuß von Hydrazin bzw. Hydrazin-hydrat bei Temperaturen zwischen 0 und 200°C, z.B. bei Temperaturen zwischen 20 und 150°C, vorzugsweise jedoch bei der Siedetemperatur des Reaktionsgemisches, und gegebenenfalls in Gegenwart einer Säure als Kondensationsmittel wie Schwefelsäure oder p-Toluolsulfonsäure durchgeführt. Die Umsetzung kann jedoch auch ohne Lösungsmittel durchgeführt werden.

e) Zur Herstellung von Verbindungen der allgemeinen Formel I, in der $R_2$ eine über eine Methylengruppe gebundene Hydroxycarbonyl-oder Alkoxycarbonylgruppe darstellt:

Umsetzung einer Verbindung der allgemnen Formel

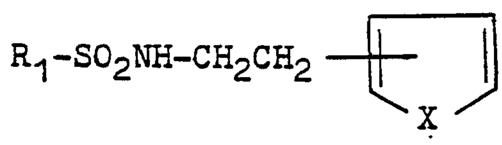

,(VIII)

in der

$R_1$ und X wie eingangs definiert sind, mit einem Diazoessigester der allgemeinen Formel

$N_2CH\text{-}COOR_8$ ,(IX)

in der

$R_8$ eine Alkylgruppe mit 1 bis 3 Kohlenstoffatomen darstellt, und gegebenenfalls anschließende Hydrolyse.

Die Umsetzung wird vorzugsweise in einem geeigneten Lösungsmittel wie Ethylenchlorid, Tetrahydrofuran oder Dioxan zweckmäßigerweise unter Schutzgas und in Gegenwart eines Metalles wie Kupferpulver oder eines Schwermetallsalzes wie Kupfer(I)chlorid oder Kupfer(II)chlorid bei Temperaturen zwischen 25 und 100°C, vorzugsweise bei der Siedetemperatur des Reaktionsgemisches durchgeführt. - Die anschließende Hydrolyse wird vorzugsweise in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches, durchgeführt.

Erhält man erfindungsgemäß eine Verbindung der allgemeinen Formel I, in der $R_2$ eine Hydroxycarbonylgruppe enthält, so kann diese mittels Veresterung in eine entsprechende Alkoxycarbonylverbindung übergeführt werden.

Die nachträgliche Veresterung wird zweckmäßigerweise in einem Lösungsmittel, z.B. in einem Überschuß des eingesetzten Alkohols wie Methanol, Äthanol oder Isopropanol, in Gegenwart eines die Säure aktivierenden Mittels wie Thionylchlorid oder Chlorwasserstoffgas bei Temperaturen zwischen 0 und 180°C, vorzugsweise jedoch bei der Siedetemperatur des Reaktionsgemisches, durchgeführt.

Die erhaltenen Verbindungen der allgemeinen Formel I können, falls $R_2$ ein optisch aktives Kohlenstoffatom enthält, ferner in ihre Enantiomeren aufgetrennt werden. So lassen sich die erhaltenen Verbindungen der allgemeinen Formel I, welche nur ein optisch aktives Zentrum enthalten, nach an sich bekannten Methoden (siehe Alinger N. L. und Elich W. L. in "Topics in Stereochemistry", Vol. 6, Wiley Interscience, 1971) in ihre optischen Antipoden auftrennen, z.B. durch Umkristallisieren aus einem optisch aktiven Lösungsmittel oder durch Umsetzen mit einer, mit der racemischen Verbindung Salze bildenden optisch aktiven Substanz, insbesondere Basen, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Basen sind z.B. die D-und L-Formen von α-Phenyl-äthylamin oder Cinchonidin.

Desweiteren lassen sich die so erhaltenen neuen Verbindungen der allgemeinen Formel I, falls diese eine Carboxygruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Äthanolamin, Diäthanolamin und Triäthanolamin in Betracht.

Wie bereits eingangs erwähnt, weisen die neuen Verbindungen und deren physiologisch verträgliche Additionssalze mit anorganischen oder organischen Basen wertvolle pharmakologische Eigenschaften auf, insbesondere antithrombotische Wirkungen und eine Hemmwirkung auf die Plättchenaggregation. Außerdem stellen sie auch Thromboxanantagonisten dar. Ferner weisen die neuen Pyridazinone der allgemeinen Formel I auf Grund ihrer Hemmwirkung auf die Phosphodiesterase eine Hemmwirkung auf die Tumormetastasierung auf.

Beispielsweise werden die neuen Verbindungen

A = 4-[2-(2-Benzolsulfonylamino-äthyl)-1-methylpyrrol-5-yl]-4-oxo-buttersäure,

B = 4-[2-(2-(p-Chlorbenzolsulfonylamino)-äthyl)-1-methylpyrrol-5-yl]-4-oxobuttersäure,

C = 4-[2-(2-Thiophen-2-yl-sulfonylamino-äthyl)-1-methylpyrrol-5-yl]-4-oxobuttersäure,

D = 4-[2-(2-(p-Fluorbenzolsulfonylamino)-äthyl)-1-methylpyrrol-5-yl]-4-oxobuttersäure und

E = 6-[2-(2-Benzolsulfonylamino-äthyl)-1-methylpyrrol-5-yl]-4,5-dihydro-3(2H)pyridazinon auf ihre biologischen Eigenschaften wie folgt geprüft:

1. Antithrombotische Wirkung

Methodik

Die Thrombozytenaggregation wird nach der Methode von BORN und CROSS (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37°C inkubiert.

Aus den erhaltenen Meßzahlen wird graphisch eine $EC_{50}$ berechnet, die sich auf eine 50%ige Änderung der "optical density" im Sinne einer Aggregationshemmung bezieht.

Die nachfolgende Tabelle enthält die gefundenen Ergebnisse:

| Substanz | $EC_{50}$ [Mol/l] |
|---|---|
| A | $3,5 \times 10^{-6}$ |
| B | $4,0 \times 10^{-7}$ |
| C | $3,9 \times 10^{-7}$ |
| D | $3,6 \times 10^{-7}$ |
| E | $2,9 \times 10^{-6}$ |

2. Akute Toxizität

Die akute Toxizität der zu untersuchenden Substanzen wurde orientierend an Gruppen von je 10 Mäusen nach oraler Gabe einer Einzeldosis bestimmt (Beobachtungszeit: 14 Tage):

| Substanz | Orientierende akute Toxizität |
| --- | --- |
| A | 500 mg/kg (0 von 10 Tieren gestorben) |
| B | 500 mg/kg (0 von 10 Tieren gestorben) |
| C | 500 mg/kg (0 von 10 Tieren gestorben) |
| D | 500 mg/kg (0 von 10 Tieren gestorben) |
| E | 500 mg/kg (0 von 10 Tieren gestorben) |

Aufgrund ihrer pharmakologischen Eigenschaften eignen sich die neuen Verbindungen und deren physiologisch verträgliche Additionssalze zur Behandlung und zur Prophylaxe thrombo-embolischer Erkrankungen wie Coronarinfarkt, Cerebralinfarkt, sogen. transient ischaemic attacks, Amaurosis fugax, zur Prophylaxe der Arteriosklerose und zur Metastasenprophylaxe.

Die zur Erzielung einer entsprechenden Wirkung erforderliche Dosierung beträgt zweckmäßigerweise zwei-bis viermal täglich 0,3 bis 4 mg/kg Körpergewicht, vorzugsweise 0,3 bis 2 mg/kg Körpergewicht. Hierzu lassen sich die erfindungsgemäß hergestellten Verbindungen der allgemeinen Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Was ser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten.

Ein weiterer Gegenstand der vorliegenden Erfindung sind die neuen Zwischenprodukte der allgemeinen Formel

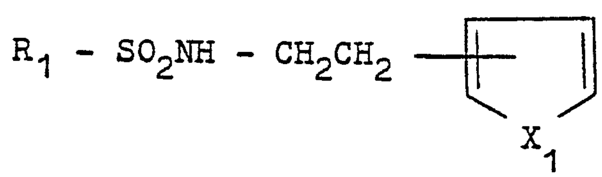

,(X)

in der

$R_1$ wie eingangs definiert ist und

$X_1$ eine gegebenenfalls durch eine Alkylgruppe mit 1 bis 3 Kohlenstoffatomen substituierte Iminogruppe oder ein Sauerstoffatom darstellt.

Die neuen Verbindungen der allgemeinen Formel VIII erhält man durch Acylierung einer Verbindung der allgemeinen Formel

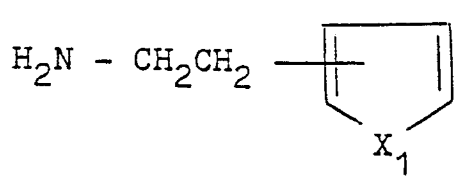

,(XI)

in der
X1 wie eingangs definiert ist, mit einem Phenylsulfonsäurederivat der allgemeinen Formel

$R_1$ -$SO_2Y_1$· ,(III)

in der
$R_1$ wie eingangs difiniert ist und
$Y_1$ eine nucleophile Austrittsgruppe wie ein Halogentom oder eine Alkoxygruppe, z.B. ein Chlor-oder Bromatom, eine Methoxy-oder Äthoxygruppe, darstellt.

Die Umsetzung wird vorzugsweise in einem Lösungsmittel wie Methanol, Äthanol, Wasser/Methanol, Dioxan, Tetrahydrofuran oder Chloroform gegebenenfalls in Gegenwart eines säurebindenden Mittels wie Kaliumcarbonat, Triäthylamin oder Pyridin, wobei die beiden letzteren auch als Lösungsmittel verwendet werden können, zweckmäßigerweise bei Temperaturen zwischen 0 und 50°C, vorzugsweise jedoch bei Raumtemperatur, durchgeführt.

Die als Ausgangsstoffe verwendeten Verbindungen der allgemeinen Formeln II bis XI erhält man nach literaturbekannten Verfahren bzw. sind literaturbekannt.

Eine als Ausgangsstoff verwendete Verbindung der allgemeinen Formel II erhält man aus einer entsprechenden N-Acylaminoethyl-verbindung durch Acylierung nach Friedel-Craft, anschließende Entacylierung und gegebenenfalls anschließende Reduktion, Hydrolyse und/oder Veresterung.

Die als Ausgangsstoffe verwendeten Verbindungen der allgemeinen Formeln IV, V, VII und VIII erhält man durch Umsetzung einer entsprechenden Aminoverbindung mit einem entsprechenden Sulfonylhalogenid.

Die nachfolgenden Beispiele sollen die Erfindung näher erläutern:

Beispiel A

2-[2-(p-Chlorbenzolsulfonylamino)-äthyl]-1-methylpyrrol

Zu einer Lösung von 4,96 g 2-(2-Aminoäthyl)-1-methylpyrrol in 20 ml Dioxan gibt man unter Rühren 10 ml einer gesättigten Kaliumcarbonatlösung. Dann läßt man bei 0°C eine Lösung von 10,1 g p-Chlorbenzolsulfonylchlorid in 30 ml Dioxan zutropfen. Anschließend wird 3 Stunden bei Raumtemperatur gerührt, dann saugt man den Niederschlag ab, wäscht mit Äther und trennt die organische Phase ab. Nach Trocknen über Natriumsulfat erhält man 10 g Rohprodukt, das über eine Kieselgelsäule mit Methylenchlorid/Cyclohexan (9:1) chromatographiert wird. Ausbeute: 6,6 g (55 % der Theorie),
Schmelzpunkt: 73-75°C
$C_{13}H_{15}ClN_2O_2S$ (298,81)

| | | | |
|---|---|---|---|
| Ber.: | C 52,25 | H 5,05 | N 9,38 |
| Gef.: | C 52,70 | H 4,95 | N 9,16 |

Beispiel B

2-(2-Benzolsulfonylamino-äthyl)-1-methylpyrrol

Hergestellt aus 2-(2-Aminoäthyl)-1-methylpyrrol und Benzolsulfonylchlorid analog Beispiel A. Ausbeute: 76 % der Theorie,
Schmelzpunkt: 63-65°C
$C_{13}H_{16}N_2O_2S$ (264,4)

| | | | |
|---|---|---|---|
| Ber.: | C 59,07 | H 6,10 | N 10,60 |
| Gef.: | C 59,22 | H 6,97 | N 10,62 |

Beispiel C

2-[2-(p-Fluorbenzolsulfonylamino)-äthyl]-1-methylpyrrol

Hergestellt aus 2-(2-Aminoäthyl)-1-methylpyrrol und p-Fluorbenzolsulfonylchlorid analog Beispiel A. Ausbeute: 89 % der Theorie,
Schmelzpunkt: 92-95°C
$C_3H_{15}FN_2O_2S$ (282,35)

| | | | |
|---|---|---|---|
| Ber.: | C 55,30 | H 5,35 | N 9,92 |
| Gef.: | C 55,15 | H 5,05 | N 9,84 |

Beispiel D

2-(2-Benzylsulfonylamino-äthyl)-1-methylpyrrol

Hergestellt aus 2-(2-Aminoäthyl)-1-methylpyrrol und Benzylsulfonylchlorid analog Beispiel A. Ausbeute: 74 % der Theorie,
Schmelzpunkt 91-93°C
$C_{14}H_{18}N_2O_2S$ (278,4)

| | | | |
|---|---|---|---|
| Ber.: | C 60,41 | H 6,52 | N 10,07 |
| Gef.: | C 60,70 | H 6,72 | N 10,15 |

Beispiel E

2-[2-(2-Thienylsulfonylamino)-äthyl]-1-methylpyrrol

Hergestellt aus 2-(2-Aminoäthyl)-1-methylpyrrol und 2-Thienylsulfonylchlorid analog Beispiel A. Ausbeute: 89 % der Theorie,
Schmelzpunkt: 63-65°C
$C_{11}H_{14}N_2O_2S_2$ (270,38)

| | | | |
|---|---|---|---|
| Ber.: | C 48,87 | H 5,22 | N 10,36 |
| Gef.: | C 49,62 | H 5,13 | N 10,30 |

Beispiel F

2-[2-(2,4,5-Trichlorbenzolsulfonylamino)-äthyl]-1-methylpyrrol

Hergestellt aus 2-(2-Aminoäthyl)-1-methylpyrrol und 2,4,5-Trichlorbenzolsulfonylchlorid analog Beispiel A. Ausbeute: 88 % der Theorie,
Schmelzpunkt: 87-90°C
$C_{13}H_{13}Cl_3N_2O_2S$ (367,7)

| | | | |
|---|---|---|---|
| Ber.: | C 42,46 | H 3,56 | N 7,62 |
| Gef.: | 43,10 | 3,65 | 7,46 |

Beispiel G

2-[2-(p-Toluolsulfonylamino)-äthyl]-1-methylpyrrol

Hergestellt aus 2-(2-Aminoäthyl)-1-methylpyrrol und p-Toluolsulfonylchlorid analog Beispiel A. Ausbeute: 73 % der Theorie,
Schmelzpunkt: 63-65°C
$C_{14}H_{18}N_2O_2S$ (278,4)

| | | | |
|---|---|---|---|
| Ber.: | C 60,39 | H 6,51 | N 10,06 |
| Gef.: | 60,40 | 6,65 | 9,91 |

Beispiel H

2-[2-(p-Acetamidobenzolsulfonylamino)-äthyl]-1-methylpyrrol

Hergestellt aus 2-(2-Aminoäthyl)-1-methylpyrrol und p-Acetamidobenzolsulfonylchlorid analog Beispiel A. Ausbeute: 80 % der Theorie,
Schmelzpunkt: 123-125°C
$C_{15}H_{19}N_3O_3S$ (321,4)

| | | | |
|---|---|---|---|
| Ber.: | C 56,05 | H 5,95 | N 13,07 |
| Gef.: | C 55,87 | H 5,87 | N 12,35 |

Beispiel I

2-[2-(p-Nitrobenzolsulfonylamino)-äthyl]-1-methylpyrrol

Hergestellt aus 2-(2-Aminoäthyl)-1-methylpyrrol und p-Nitrobenzolsulfonylchlorid analog Beispiel A. Ausbeute: 78 % der Theorie,
Schmelzpunkt: 102°C
$C_{13}H_{15}O_4N_3S$ (309,35)

| | | | |
|---|---|---|---|
| Ber.: | C 50,46 | H 4,88 | N 13,59 |
| Gef.: | 50,60 | 4,86 | 13,54 |

Beispiel K

2-[2-(2-Methoxybenzolsulfonylamino)-äthyl]-1-methylpyrrol

Hergestellt aus 2-(2-Aminoäthyl)-1-methylpyrrol und 2-Methoxybenzolsulfonylchlorid analog Beispiel A. Ausbeute: 75 % der Theorie,
Schmelzpunkt: Öl
$C_{14}H_{18}O_3N_2S$ (294,3)

| | | | |
|---|---|---|---|
| Ber.: | C 57,13 | H 6,16 | N 9,51 |
| Gef.: | C 56,41 | H 6,10 | N 9,10 |

Beispiel L

2-[2-(2,5-Dichlorbenzolsulfonylamino)-äthyl]-1-methylpyrrol

Hergestellt aus 2-(2-Aminoäthyl)-1-methylpyrrol und 2,5-Dichlorbenzolsulfonylchlorid analog Beispiel A. Ausbeute: 60 % der Theorie,
Schmelzpunkt: 84-86°C
$C_{13}H_{14}C_{12}N_2O_2S$ (333,25)

| | | | |
|---|---|---|---|
| Ber.: | C 46,85 | H 4,24 | N 8,41 |
| Gef.: | C 46,84 | H 4,26 | N 8,54 |

Beispiel M

2-(2-Benzolsulfonylamino-äthyl)-furan

3,2 g 2-(2-Aminoäthyl)-furan werden in 45 ml Pyridin gelöst und bei 5°C mit einer Lösung von 8,8 g Benzolsulfonsäurechlorid in 50 ml Pyridin versetzt. Dann läßt man bei Raumtemperatur über Nacht rühren, engt das Reaktionsprodukt ein, nimmt den Rückstand in Wasser auf und extrahiert mit Methylenchlorid. Nach Trocknen der organischen Phase über Magnesiumsulfat engt man die Lösung ein und chromatographiert über eine Kieselgelsäule mit Ethylenchlorid. Ausbeute: 78 % der Theorie,
Schmelzpunkt: Harzartiges Produkt
$C_{12}H_{13}NO_3S$ (251,31)

| | | | |
|---|---|---|---|
| Ber.: | C 57,35 | H 5,21 | N 5,58 |
| Gef.: | C 57,53 | H 5,20 | N 5,76 |

Beispiel 1

4-[2-(2-(p-Chlorbenzolsulfonylamino)-äthyl)-1-methylpyrrol-5-yl]-4-oxobuttersäure-methylester

Zu einer Lösung von 3,9 g 2-[2-(p-Chlorbenzolsulfonylamino)-äthyl]-1-methylpyrrol in 35 ml trockenem Ethylenchlorid gibt man 2,7 g Carbomethoxypropionylchlorid und rührt 3 Stunden bei 60°C. Anschließend engt man die Lösung ein und chromatographiert den Rückstand über eine Kieselgelsäule mit Chloroform/Essigester (7:3). Ausbeute: 45 % der Theorie,
Schmelzpunkt: sintert ab 60°C
$C_{18}H_{21}ClN_2O_5S$ (412,92)

| | | | |
|---|---|---|---|
| Ber.: | C 52,37 | H 5,13 | Cl 8,58 |
| Gef.: | C 53,64 | H 5,68 | Cl 8,25 |

Beispiel 2

4-[2-(2-Benzolsulfonylamino-äthyl)-1-methylpyrrol-5-yl]-4-oxobuttersäure-methylester

Hergestellt aus 2-(2-Benzolsulfonylaminoethyl)-1-methylpyrrol und Carbomethoxypropionylchlorid analog Beispiel 1. Ausbeute: 43 % der Theorie,
Schmelzpunkt: 89-91°C
$C_{18}H_{22}N_2O_5S$ (378,47)

| | | | |
|---|---|---|---|
| Ber.: | C 57,13 | H 5,86 | N 7,40 |
| Gef.: | C 57,11 | H 5,73 | N 7,30 |

Beispiel 3

4-[2-(2-(p-Fluorbenzolsulfonylamino)-äthyl)-1-methylpyrrol-5-yl]-4-oxobuttersäure-methylester

Hergestellt aus 2-[2-(p-Fluorbenzolsulfonylamino)-äthyl]-1-methylpyrrol und Carbomethoxypropionylchlorid analog Beispiel 1. Ausbeute: 30 % der Theorie,
Schmelzpunkt: 85-88°C
$C_{18}H_{21}FN_2O_5S$ (396,46)

| | | | |
|---|---|---|---|
| Ber.: | C 54,54 | H 5,34 | N 7,07 |
| Gef.: | C 54,30 | H 5,20 | N 7,19 |

Beispiel 4

4-[2-(2-Benzolsulfonylamino-äthyl)-1-methylpyrrol-5-yl]-4-oxobuttersäure-methylester

Hergestellt aus 2-(2-Benzolsulfonylamino-äthyl)-1-methylpyrrol und Carbomethoxypropionylchlorid analog Beispiel 1. Ausbeute: 34 % der Theorie,
Schmelzpunkt: Harzartiges Produkt
$C_{19}H_{24}N_2O_2S$ (392,49)

| | | | |
|---|---|---|---|
| Ber.: | C 58,14 | H 6,16 | S 8,17 |
| Gef.: | C 58,00 | H 6,07 | S 8,08 |

Beispiel 5

4-[2-(2-Thiophen-2-ylsulfonylamino-äthyl)-1-methylpyrrol-5-yl]-4-oxobuttersäure-methylester

Hergestellt aus 2-(2-Thiophen-2-yl-sulfonylamino-äthyl)-1-methylpyrrol und Carbomethoxypropionylchlorid analog Beispiel 1. Ausbeute: 40 % der Theorie,
Schmelzpunkt: Harzartiges Produkt
$C_{16}H_{20}N_2O_5S_2$ (384,49)

| | | | |
|---|---|---|---|
| Ber.: | C 49,98 | H 5,24 | N 7,29 |
| Gef.: | C 49,74 | H 5,39 | N 7,55 |

Beispiel 6

4-[2-(2-(2,4,5-Trichlorbenzolsulfonylamino)-äthyl)-1-methylpyrrol-5-yl]-4-oxobuttersäure-methylester

Hergestellt aus 2-[2-(2,4,5-Trichlorbenzolsulfonylamino)äthyl]-1-methylpyrrol und Carbomethoxypropionylchlorid analog Beispiel 1. Ausbeute: 57 % der Theorie,
Schmelzpunkt: Harzartiges Produkt
$C_{18}H_{19}Cl_3N_2O_5S$ (481,81)

| | | | |
|---|---|---|---|
| Ber.: | C 44,88 | H 3,95 | S 6,65 |
| Gef.: | C 45,12 | H 4,34 | S 6,81 |

Beispiel 7

4-[2-(2-(p-Toluolsulfonylamino)-äthyl)-1-methylpyrrol-5-yl]-4-oxobuttersäure-methylester

Hergestellt aus 2-[2-(p-Toluolsulfonylamino)-äthyl]-1-methylpyrrol und Carbomethoxypropionylchlorid analog Beispiel 1. Ausbeute: 35 % der Theorie,
$C_{19}H_{17}O_5N_2S$ (385,42)

| | | | |
|---|---|---|---|
| Ber.: | C 59,21 | H 4,44 | S 8,32 |
| Gef.: | C 59,66 | H 5,07 | S 8,84 |

Beispiel 8

4-[2-(2-(p-Acetamidobenzolsulfonylamino)-äthyl)-1-methylpyrrol-5-yl]-4-oxobuttersäure-methylester

Hergestellt aus 2-[2-(p-Acetamidobenzolsulfonylamino)-äthyl]-1-methylpyrrol und Carbomethoxypropionylchlorid analog Beispiel 1. Ausbeute: 8 % der Theorie,
Schmelzpunkt: 130-131 °C
$C_{20}H_{22}N_3O_6S$ (435,5)

| | | | |
|---|---|---|---|
| Ber.: | C 55,15 | H 5,78 | S 7,36 |
| Gef.: | C 56,48 | H 5,52 | S 7,48 |

Beispiel 9

4-[2-(2-(p-Nitrobenzolsulfonylamino)-äthyl)-1-methylpyrrol-5-yl]-4-oxobuttersäure-methylester

Hergestellt aus 2-(2-(p-Nitrobenzolsulfonylamino)-äthyl]-1-methylpyrrol und Carbomethoxypropionylchlorid analog Beispiel 1. Ausbeute: 44 % der Theorie,
Schmelzpunkt: 118-120°C
$C_{18}H_{21}N_3O_7S$ (423,4)

| | | | |
|---|---|---|---|
| Ber.: | C 51,06 | H 4,99 | N 9,92 |
| Gef.: | C 50,80 | H 4,96 | N 9,97 |

Beispiel 10

4-[2-(2-(o-Methoxybenzolsulfonylamino)-äthyl)-1-methylpyrrol-5-yl]-4-oxobuttersäure-methylester

Hergestellt aus 2-[2-(o-Methoxybenzolsulfonylamino)-äthyl]-1-methylpyrrol und Carbomethoxypropionylchlorid analog Beispiel 1. Ausbeute: 53 % der Theorie,
Schmelzpunkt: Harz
$C_{19}H_{24}N_2O_6S$ (380,5)

| | | | |
|---|---|---|---|
| Ber.: | C 55,86 | H 5,92 | N 6,85 |
| Gef.: | C 56,40 | H 5,98 | N 6,67 |

Beispiel 11

4-[2-(2-(p-Chlorbenzolsulfonylamino)-äthyl)-1-methylpyrrol-5-yl]-4-oxobuttersäure

1,03 g 4-[2-(2-(p-Chlorbenzolsulfonylamino)-äthyl)-1-methylpyrrol-5-yl]-4-oxobuttersäure-methylester werden in 15 ml 1N Natronlauge gegeben und 3 Stunden bei 50°C gerührt. Anschließend stellt man die Lösung mit 8 ml 2N Salzsäure sauer und saugt den Niederschlag ab. Ausbeute: 0,5 g (50 % der Theorie),
Schmelzpunkt: 127-130°C

$C_{17}H_{19}ClN_2O_5S$ (398,89)

| | | | |
|---|---|---|---|
| Ber.: | C 51,18 | H 4,80 | N 7,03 |
| Gef.: | C 51,20 | H 4,36 | N 6,90 |

Beispiel 12

4-[2-(2-Benzolsulfonylamino-äthyl)-1-methylpyrrol-5-yl]-4-oxobuttersäure

Hergestellt aus 4-[2-(2-Benzolsulfonylamino-äthyl)-1-methylpyrrol-5-yl]-4-oxobuttersäure-methylester analog Beispiel 11. Ausbeute: 77 % der Theorie,
Schmelzpunkt: 120-122°C
C1H12$N_2O_5S$ (364,4)

| | | | |
|---|---|---|---|
| Ber.: | C 56,03 | H 5,53 | N 7,69 |
| Gef.: | 55,90 | 5,33 | 7,43 |

Beispiel 13

4-[2-(2-(p-Fluorbenzolsulfonylamino)-äthyl)-1-methylpyrrol-5-yl]-4-oxobuttersäure

Hergestellt aus 4-[2-(2-(p-Fluorbenzolsulfonylamino)-äthyl)-1-methylpyrrol-5-yl]-4-oxobuttersäure-methylester analog Beispiel 11. Ausbeute: 92 % der Theorie,
Schmelzpunkt: 142-143°C
$C_{17}H_{19}FN_2O_5S$ (382,43)

| | | | |
|---|---|---|---|
| Ber.: | C 53,39 | H 5,01 | N 7,32 |
| Gef.: | C 53,57 | H 4,92 | N 6,86 |

Beispiel 14

4-[2-(2-Benzylsulfonylamino-äthyl)-1-methylpyrrol-5-yl]-4-oxobuttersäure

Hergestellt aus 4-[2-(2-Benzylsulfonylamino-äthyl)-1-methylpyrrol-5-yl]-4-oxobuttersäure-methylester analog Beispiel 11. Ausbeute: 70 % der Theorie,
Schmelzpunkt: 156-159°C
$C_{18}H_{22}N_2O_5S$ (378,47)

| | | | |
|---|---|---|---|
| Ber.: | C 57,13 | H 5,86 | N 7,40 |
| Gef.: | C 57,20 | H 5,72 | N 7,11 |

Beispiel 15

4-[2-(2-Thiophen-2-yl-sulfonylamino-äthyl)-1-methylpyrrol-5-yl]-4-oxobuttersäure

Hergestellt aus 4-[2-(2-Thiophen-2-yl-sulfonylamino-äthyl)-1-methylpyrrol-5-yl]-4-oxobuttersäure-methylester analog Beispiel 11. Ausbeute: 41 % der Theorie,
Schmelzpunkt: 135-137°C
$C_{15}H_{18}N_2O_5S_2$ (370,46)

| | | | |
|---|---|---|---|
| Ber.: | C 48,63 | H 4,90 | N 7,56 |
| Gef.: | C 48,36 | H 4,83 | N 7,60 |

Beispiel 16

4-[2-(2-(2,4,5-Trichlorbenzolsulfonylamino)-äthyl)-1-methylpyrrol-5-yl]-4-oxobuttersäure

Hergestellt aus 4-[2-(2-(2,4,5-Trichlorbenzolsulfonylamino)-äthyl)-1-methylpyrrol-5-yl]-4-oxobuttersäure-methylester analog Beispiel 11. Ausbeute: 78 % der Theorie,
Schmelzpunkt: 152-155°C
$C_{17}H_{17}Cl_3N_2O_5S$ (467,79)

| | | | |
|---|---|---|---|
| Ber.: | C 43,65 | H 3,66 | N 5,99 |
| Gef.: | C 43,42 | H 3,42 | N 5,98 |

Beispiel 17

4-[2-(2-(p-Toluolsulfonylamino)-äthyl)-1-methylpyrrol-5-yl]-4-oxo-buttersäure

Hergestellt aus 4-[2-(2-(p-Toluolsulfonylamino)-äthyl)-1-methylpyrrol-5-yl]-4-oxobuttersäure-methylester analog Beispiel 11. Ausbeute: 86 % der Theorie,
Schmelzpunkt: 195-198°C
$C_{18}H_{22}N_2O_5S$ (378,5)

| | | | |
|---|---|---|---|
| Ber.: | C 57,13 | H 5,86 | N 7,40 |
| Gef.: | C 57,20 | H 5,87 | N 7,36 |

Beispiel 18

4-[2-(2-(p-Acetamido-benzolsulfonylamino)-äthyl)-1-methylpyrrol-5-yl]-4-oxobuttersäure

Hergestellt aus 4-[2-(2-(p-Acetamido-benzolsulfonylamino)-äthyl)-1-methylpyrrol-5-yl]-4-oxobuttersäure-methylester analog Beispiel 11. Ausbeute: 24 % der Theorie,
Schmelzpunkt: 124-126°C $C_{19}H_{23}N_3O_6S$ (421,5)

| | | | |
|---|---|---|---|
| Ber.: | C 54,14 | H 5,50 | N 9,97 |
| Gef.: | C 53,03 | H 5,50 | N 10,62 |

Beispiel 19

4-[2-(2-(p-Nitrobenzolsulfonylamino)-äthyl)-1-methylpyrrol-5-yl]-4-oxobuttersäure

Hergestellt aus 4-[2-(2-(p-Nitrobenzolsulfonylamino)-äthyl)-1-methylpyrrol-5-yl]-4-oxobuttersäure-methylester analog Beispiel 11. Ausbeute: 55 % der Theorie,
Schmelzpunkt: 181°C
$C_{17}H_{19}N_3O_7S$ (409,4)

| | | | |
|---|---|---|---|
| Ber.: | C 49,87 | H 4,68 | N 10,26 |
| Gef.: | C 49,50 | H 4,55 | N 10,33 |

Beispiel 20

4-[2-(2-(2,5-Dichlorbenzolsulfonylamino)-äthyl)-1-methylpyrrol-5-yl]-4-oxobuttersäure

Zu einer Suspension von 2,7 g Aluminiumchlorid in 20 ml trockenem Ethylenchlorid gibt man bei -5°C 1,1 g Bernsteinsäureanhydrid in 50 ml Ethylenchlorid. Anschließend gibt man 3,3 g 2-[2-(2,5-Dichlorbenzolsulfonylamino)-äthyl]-1-methylpyrrol in 25 ml Ethylenchlorid zu und rührt 3 Stunden bei Raumtemperatur. Dann gibt man Eiswasser zu, trennt die organische Phase ab, schüttelt sie mit 2%iger Natronlauge und säuert die wässrige Phase an. Das ausgefallene harzartige Produkt wird gesammelt. Ausbeute: 0,6 g (14 % der Theorie),
Schmelzpunkt: Harz
$C_{17}H_{18}Cl_2N_2O_5S$

| | | | |
|---|---|---|---|
| Ber.: | C 47,12 | H 4,19 | Cl 16,37 |
| Gef.: | C 47,40 | H 4,29 | Cl 16,63 |

Beispiel 21

4-[2-(2-Benzolsulfonylamino-äthyl)-furan-5-yl]-4-oxobuttersäure

Hergestellt aus 2-(2-Benzolsulfonylamino-äthyl)-furan und Bernsteinsäureanhydrid analog Beispiel 20. Ausbeute: 65 % der Theorie,
Schmelzpunkt: 126-129°C
$C_{16}H_{17}NO_6S$ (351,40)

| | | | |
|---|---|---|---|
| Ber.: | C 54,69 | H 4,88 | N 3,99 |
| Gef.: | C 54,77 | H 4,69 | N 3,92 |

Beispiel 22

4-[2-(2-Benzolsulfonylamino-äthyl)-thiophen-5-yl]-4-oxobuttersäure

Hergestellt aus 2-(2-Benzolsulfonylamino-äthyl)-thiophen und Bernsteinsäureanhydrid analog Beispiel 20. Ausbeute: 62 % der Theorie,
Schmelzpunkt: 140-142°C
$C_{16}H_{17}NO_3S_2$ (367,5)

| | | | |
|---|---|---|---|
| Ber.: | C 52,30 | H 4,66 | N 3,81 |
| Gef.: | C 52,36 | H 4,79 | N 3,86 |

Beispiel 23

6-[2-(2-Benzolsulfonylamino-äthyl)-1-methylpyrrol-5-yl]-4,5-di hydro-3(2H)pyridazinon

1,46 g 4-[2-(2-Benzolsulfonylamino-äthyl)-1-methylpyrrol-5-yl]-4-oxobuttersäure werden in 10 ml Eisessig suspendiert, mit 2,2 g Hydrazinhydrat versetzt und 6 Stunden am Rückfluß erhitzt. Anschließend wird die Lösung eingeengt, das Reaktionsprodukt mit Wasser versetzt und mit 2N Ammoniak basisch gestellt. Der ausfallende Niederschlag wird im heißen Methanol gelöst, von unlöslichem Niederschlag abfiltriert und eingeengt. Ausbeute: 0,72 g (50 % der Theorie),
Schmelzpunkt: 180-182°C
$C1H_{20}N_4O_3S$ (360,4)

| | | | |
|---|---|---|---|
| Ber.: | C 56,65 | H 5,59 | N 15,54 |
| Gef.: | C 56,33 | H 5,52 | N 15,75 |

Beispiel 24

6-[2-(2-(p-Fluorbenzolsulfonylamino)-äthyl)-1-methylpyrrol-5-yl]-4,5-dihydro-3(2H)pyridazinon

Hergestellt aus 4-[2-(2-(p-Fluorbenzolsulfonylamino)-äthyl)-1-methylpyrrol-5-yl]-4-oxobuttersäure und Hydrazinhydrat analog Beispiel 23. Ausbeute: 29 % der Theorie,
Schmelzpunkt: 201 °C
$C_{17}H_{19}N_4O_3FS$ (378,4)

| | | | |
|---|---|---|---|
| Ber.: | C 53,96 | H 5,06 | N 14,86 |
| Gef.: | 53,74 | 5,35 | 14,10 |

Beispiel 25

6-[2-(2-Benzolsulfonylamino-äthyl)-furan-5-yl]-4,5-dihydro-3(2H)-pyridazinon

Hergestellt aus 4-[2-(2-Benzolsulfonylamino-äthyl)-furan-5-yl]-4-oxobuttersäure und Hydrazinhydrat analog Beispiel 23. Ausbeute: 85 % der Theorie,
Schmelzpunkt: 174-176°C
$C_{16}H_{17}N_3O_4S$ (347,4)

| | | | |
|---|---|---|---|
| Ber.: | C 55,31 | H 4,93 | N 12,09 |
| Gef.: | C 55,20 | H 5,06 | N 12,36 |

Beispiel 26

6-[2-(2-Benzolsulfonylamino-äthyl)-thiophen-5-yl]-4,5-dihydro-3(2H)-pyridazinon

Hergestellt aus 4-[2-(2-Benzolsulfonylamino-äthyl)-thiophen-5-yl]-4-oxobuttersäure und Hydrazinhydrat analog Beispiel 23. Ausbeute: 64 % der Theorie,
Schmelzpunkt: 140°C
$C_{15}H_{17}N_3O_2S_2$ (351,5)

| | | | |
|---|---|---|---|
| Ber.: | C 51,25 | H 4,87 | N 11,95 |
| Gef.: | C 51,71 | H 4,71 | N 11,78 |

Beispiel 27

4-[2-(2-Benzylsulfonylamino-äthyl)-thiophen-5-yl)-4-oxobuttersäure

a) 4-[2-(2-Acetylamino-äthyl)-thiophen-5-yl]-4-oxobuttersäure

Hergestellt analog Beispiel 23 aus 2-(2-Acetylamino-äthyl)-thiophen und Bernsteinsäureanhydrid. Ausbeute: 41 % der Theorie,
Schmelzpunkt: 116-118°C
$C_{12}H_{15}NO_4S$ (269,3)

| | | | |
|---|---|---|---|
| Ber.: | C 53,26 | H 5,61 | N 11,91 |
| Gef.: | C 53,24 | H 5,61 | N 12,03 |

b) 4-[2-(2-Amino-äthyl)-thiophen-5-yl]-4-oxobuttersäuremethylester-hydrochlorid

1,0 g 4-(2-(2-Acetylamino-äthyl)-thiophen-5-yl)-4-oxobuttersäure werden in 15 ml konz. Salzsäure und 10 ml Methanol über Nacht am Rückfluß erhitzt. Anschließend wird die Lösung eingeengt und der Rückstand getrocknet. Ausbeute: 0,6 g (57 % der Theorie),
Schmelzpunkt: 153-155°C

c) 4-[2-(2-Benzylsulfonylamino-äthyl)-thiophen-5-yl]-4-oxobuttersäure

0,6 g 4-(2-(2-Amino-äthyl)-thiophen-5-yl]-4-oxobuttersäuremethylester werden in 30 ml Pyridin gelöst und bei 0°C mit 0,95 g Benzylsulfonsäurechlorid, gelöst in 20 ml Pyridin, versetzt. Anschließend wird 3 Stunden bei Raumtemperatur gerührt, das Pyridin im Vakuum abgetrennt, der Rückstand mit Wasser versetzt und das Produkt mit Methylenchlorid extrahiert. Die vereinigten organischen Phasen werden mit Natriumsulfat getrocknet und einrotiert. Der dabei erhaltene harzartige Rückstand wird mit 20 ml 1N Natronlauge 2 Stunden bei 70°C gerührt. Diese Lösung wird filtriert und angesäuert, worauf das Produkt ausfällt. Ausbeute: 150 mg (20 % der Theorie),
Schmelzpunkt: 112-115°C
$C_{17}H_{19}NO_5S_2$ (381,5)

| | | | | |
|---|---|---|---|---|
| Ber.: | C 53,52 | H 5,02 | N 3,67 | S 16,81 |
| Gef.: | C 53,13 | H 4,80 | N 3,28 | S 16,74 |

[2-(2-(4-Fluorbenzolsulfonylamino)-ethyl)-1-methylpyrrol-5-yl]-essigsäure-äthylester

1,69 g 2-(2-(4-Fluorbenzolsulfonylamino)-ethyl)-1-methylpyrrol werden in 6 ml Ethylenchlorid gelöst und mit 0,06 g Kupfer(I)chlorid versetzt. Unter einer Stickstoffatmosphäre wird am Rückfluß erhitzt und dann langsam eine Lösung von 0,34 g Diazoessigsäure-äthylester in 6 ml Ethylenchlorid zugetropft. Nach 20 Minuten ist die Stickstoffentwicklung beendet und die Suspension wird zur Trockne einrotiert. Anschließend wird über eine Kieselgelsäule chromatographiert (Ethylenchlorid/Essigsäure-äthylester = 100/1). Ausbeute: 0,7 g (63 % der Theorie) Harz,
$C_{17}H_{21}FN_2O_4S$ (368,4)

| | | | | |
|---|---|---|---|---|
| Ber.: | C 55,42 | H 5,74 | N 7,60 | S 8,70 |
| Gef.: | C 55,22 | H 5,66 | N 7,53 | S 8,51 |

Beispiel 29

[2-(2-(4-Chlorbenzolsulfonylamino)-ethyl)-1-methylpyrrol-5-yl]-essigsäure-äthylester

Hergestellt analog Beispiel 28 aus 2-(2-(4-Chlorbenzolsulfonylamino)-ethyl-1-methylpyrrol und Diazoessigsäure-äthylester. Ausbeute: 58 % der Theorie,
Schmelzpunkt: Harz
$R_f$-Wert: 0,3 (Kieselgel-Polygram-Platten;
Laufmittel: Ethylenchlorid:Ethanol = 100:1)

Beispiel 30

[2-(2-Toluolsulfonylamino)-ethyl)-1-methylpyrrol-5-yl]-essigsäure-äthylester

Hergestellt analog Beispiel 28 aus 2-(2-Toluolsulfonylamino)-ethyl-1-methylpyrrol und Diazoessigsäure-äthylester. Ausbeute: 45 % der Theorie,
Schmelzpunkt: Harz
$R_f$ Wert: 0,2 (Kieselgel-Polygram-Platten;
Laufmittel: Ethylenchlorid:Ethanol = 100:1)

Beispiel 31

[2-(2-Benzylsulfonylamino)-ethyl)-1-methylpyrrol-5-yl]-essigsäure-äthylester

Hergestellt analog Beispiel 28 aus 2-(2-Benzylsulfonylamino)-ethyl-1-methylpyrrol und Diazoessigsäure-äthylester. Ausbeute: 38 % der Theorie,
Schmelzpunkt: Harz
$R_f$ -Wert: 0,2 (Kieselgel-Polygram-Platten;
Laufmittel: Ethylenchlorid:Ethanol = 100:1)

Beispiel 32

[2-(2-(4-Fluorbenzolsulfonylamino)-ethyl)-1-methylpyrrol-5-yl]-essigsäure

0,5 g (1,3 mMol) [2-(2-(4-Fluorbenzolsulfonylamino)-ethyl)-. 1-methylpyrrol-5-yl]-essigsäure-äthylester werden in 4,5 ml 1N Natronlauge über Nacht bei Raumtemperatur gerührt. Anschließend wird mit Ethylenchlorid ausgeschüttelt und die Wasserphase mit 1N Salzsäure angesäuert. Der ausgefallene Niederschlag wird abgesaugt und getrocknet. Ausbeute: 190 mg (43 % der Theorie),
Schmelzpunkt: 117-121 °C
$C_{15}H_{17}FN_2O_4S$ (340,4)

| | | | |
|---|---|---|---|
| Ber.: | C 52,93 | H 5,03 | N 8,23 |
| Gef.: | C 52,93 | H 5,00 | N 7,96 |

Beispiel 33

[2-(2-(4-Chlorbenzolsulfonylamino)-ethyl)-1-methylpyrrol-5-yl]-essigsäure

Hergestellt analog Beispiel 32 aus 4-[2-(2-(4-Chlorbenzolsulfonylamino)-ethyl-1-methylpyrrol-5-yl]-essigsäure-äthylester. Ausbeute: 35 % der Theorie,
Schmelzpunkt: 120-125 °C (Zers.)
$C_{15}H_{17}ClN_2O_4S$ (356,8)

| | | | | |
|---|---|---|---|---|
| Ber.: | C 50,49 | H 9,94 | N 7,85 | S 8,99 |
| Gef.: | C 50,55 | H 10,01 | N 7,98 | S 9,23 |

Beispiel 34

[2-(2-(Toluolsulfonylamino)-ethyl)-1-methylpyrrol-5-yl]-essigsäure

Hergestellt analog Beispiel 32 aus [2-(2-(Toluolsulfonylamino)-ethyl-1-methylpyrrol-5-yl]-essigsäure-äthylester. Ausbeute: 28 % der Theorie,
Schmelzpunkt: 118-123°C (Zers.)
$C_{16}H_{20}N_2O_4S$ (336,4)

| | | | | |
|---|---|---|---|---|
| Ber.: | C 57,12 | H 5,99 | N 8,33 | S 9,53 |
| Gef.: | C 57,30 | H 6,14 | N 8,45 | S 9,80 |

Beispiel 35

[2-(2-(Benzylsulfonylamino)-ethyl)-1-methylpyrrol-5-yl]-essigsäure

Hergestellt analog Beispiel 32 aus [2-(2-(Benzylsulfonylamino)-ethyl-1-methylpyrrol-5-yl]-essigsäure-äthylester. Ausbeute: 31 % der Theorie,
Schmelzpunkt: 95-100°C (Zers.)
$C_{16}H_{20}N_2O_4S$ (336,4)

| | | | | |
|---|---|---|---|---|
| Ber.: | C 57,12 | H 5,99 | N 8,33 | S 9,53 |
| Gef.: | C 57,01 | H 6,14 | N 8,45 | S 9,33 |

Beispiel I

Tabletten mit 100 mg 4-[2-(2-(p-Fluorbenzolsulfonylamino)-äthyl)-1-methyl-pyrrol-5-yl]-4-oxobuttersäure

Zusammensetzung:
1 Tablette enthält:

| | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungsverfahren:

Wirkstoff, Milchzucker und Stärke werden gemischt und mit einer wäßrigen Lösung des Polyvinylpyrrolidons gleichmäßig befeuchtet. Nach Siebung der feuchten Massen (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet. Tablettengewicht: 220 mg
Durchmesser: 9 mm, biplan mit beidseitiger Facette und einseitiger Teilkerbe.

Beispiel II

Hartgelatine-Kapseln mit 150 mg 4-[2-(2-(p-Fluorbenzolsulfonylamino)-äthyl)-1-methyl-pyrrol-5-yl]-4-oxobuttersäure

1 Kapsel enthält:

| | | |
|---|---|---|
| Wirkstoff | | 150,0 mg |
| Maisstärke getr. | ca. | 180,0 mg |
| Milchzucker pulv. | ca. | 87,0 mg |
| Magnesiumstearat | | 3,0 mg |
| | ca. | 320,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt. Kapselfüllung: ca. 320 mg
Kapselhülle: Hartgelatine-Kapsel Größe 1.

Beispiel III

Suppositorien mit 150 mg 4-[2-(2-(p-Fluorbenzolsulfonylamino)-äthyl)-1-methyl-pyrrol-5-yl]-4-oxobuttersäure

1 Zäpfchen enthält:

| | |
|---|---|
| Wirkstoff | 150,0 mg |
| Polyäthylenglykol (M.G. 1500) | 550,0 mg |
| Polyäthylenglykol (M.G. 6000) | 460,0 mg |
| Polyäthylensorbitanmonostearat | 840,0 mg |
| | 2 000,0 mg |

Herstellung:

Nach dem Aufschmelzen der Suppositorienmassen wird der Wirkstoff darin homogen verteilt und die Schmelze in vorgekühlte Formen gegossen.

Beispiel IV

Suspensionen mit 50 mg 4-[2-(2-(p-Fluorbenzolsulfonylamino)-äthyl)-1-methyl-pyrrol-5-yl]-4-oxobuttersäure
100 ml Suspension enthalten:

| | |
|---|---|
| Wirkstoff | 1,0 g |
| Carboxymethylcellulose-Na-Salz | 0,2 g |
| p-Hydroxybenzoesäuremethylester | 0,05 g |
| p-Hydroxybenzoesäurepropylester | 0,01 g |

Glycerin 5,0 g
Sorbitlösung 70%ig 50,0 g
Aroma 0,3 g
Wasser dest. ad 100 ml

Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

Beispiel V

Tabletten mit 150 mg 4-[2-(2-(p-Fluorbenzolsulfonylamino)-äthyl)-1-methyl-pyrrol-5-yl]-4-oxobuttersäure

Zusammensetzung:
1 Tablette enthält:

| | |
|---|---|
| Wirksubstanz | 150,0 mg |
| Milchzucker pulv. | 89,0 mg |
| Maisstärke | 40,0 mg |
| Kolloidale Kieselsäure | 10,0 mg |
| Polyvinylpyrrolidon | 10,0 mg |
| Magnesiumstearat | 1,0 mg |
| | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen. Das bei 45°C getrocknete Granulat wird nochmals durch dasselbe Sieb gerieben und mit der angegebenen Menge Magnesiumstearat gemischt. Aus der Mischung werden Tabletten gepreßt.

Tablettengewicht: 300 mg
Stempel: 10 mm, flach

Beispiel VI

Filmtabletten mit 75 mg 4-[2-(2-(p-Fluorbenzolsulfonylamino)-äthyl)-1-methyl-pyrrol-5-yl]-4-oxobuttersäure

1 Tablettenkern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm Maschenweite gerieben und mit der restlichen Menge Magnesiumstearat vermischt. Dieses Granulat wird auf einer Tablettiermaschine zu Tabletten mit der gewünschten Form gepreßt.

Kerngewicht: 230 mg
Stempel: 9 mm, gewölbt

Die so hergestellten Tablettenkerne werden mit einem Film überzogen, der im wesentlichen aus Hydroxypropylmethylcellulose besteht. Die fertigen Filmtabletten werden mit Bienenwachs geglänzt.

Filmtablettengewicht: 245 mg

Selbstverständlich können alle übrigen Verbindungen der allgemeinen Formel I als Wirkstoffe in den vorstehenden galenischen Zubereitungen eingesetzt werden.

**Ansprüche**

1. Neue Sulfonamido-äthylverbindungen der allgemeinen Formel

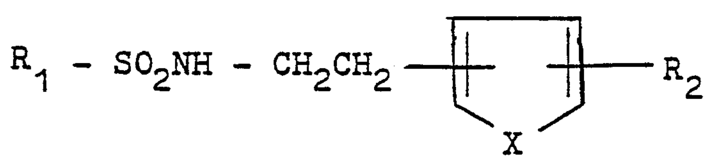

(I)

in der

$R_1$ eine gegebenenfalls durch ein Halogenatom, eine Methyl-oder Methoxygruppe mono-, di-oder trisubstituierte Phenylgruppe, wobei die Substituenten gleich oder verschieden sein können, eine Benzyl-, Nitrophenyl-, Acetamidophenyl-oder Thienylgruppe,

$R_2$ eine über eine geradkettige oder verzweigte Alkylengruppe mit 1 bis 5 Kohlenstoffatomen oder über eine Alkenylengruppe mit 2 bis 5 Kohlenstoffatomen gebundene Hydroxycarbonyl-oder Alkoxycarbonylgruppe mit insgesamt 2 bis 4 Kohlenstoffatomen, wobei jeweils eine Methylengruppe der vorstehend erwähnten Alkylen-oder Alkenylenreste, welche mit dem heterocyclischen Rest verknüpft sein muß, durch eine

Hydroxymethylen-oder Carbonylgruppe ersetzt sein kann, oder eine gegebenenfalls im Kohlenstoffgerüst durch eine Alkylgruppe mit 1 bis 3 Kohlenstoffatomen substituierte 4,5-Dihydro-pyridazin-3-on-6-yl-oder Pyridazin-3-on-6-yl-gruppe und

X eine gegebenenfalls durch eine Alkylgruppe mit 1 bis 3 Kohlenstoffatomen substituierte Iminogruppe, ein Sauerstoff-oder Schwefelatom bedeuten, deren Enantiomere und deren Salze mit anorganischen oder organischen Basen, falls $R_2$ eine Hydroxycarbonylgruppe enthält.

2. Neue Sulfonamido-äthylverbindungen der allgemeinen Formel I gemäß Anspruch 1, in der

$R_1$ eine gegebenenfalls durch eine Methyl-, Methoxy-oder Nitrogruppe substituierte Phenylgruppe, eine durch ein Fluor-, Chlor-oder Bromatom mono-, di-oder trisubstituierte Phenylgruppe und

$R_2$ eine Hydroxycarbonylmethyl-, Äthoxycarbonylmethyl-, 2-Äthoxycarbonyl-äthyl-, 3-Hydroxycarbonyl-n-propan-(1)-yl-, 3-Methoxycarbonyl-n-propan-(1)-yl-, 3-Hydroxycarbonyl-n-propan-1-on-(1)-yl-, 3-Methoxycarbonyl-n-propan-1-on-(1)-yl-oder 4,5-Dihydro-pyridazin-3-on-6-yl-gruppe und

X eine Methyliminogruppe, ein Sauerstoff-oder Schwefelatom bedeuten, deren Enantiomere und deren Salze mit anorganischen oder organischen Basen, falls $R_2$ eine Hydroxycarbonylgruppe enthält.

3. Neue Sulfonamido-äthylverbindungen der allgemeinen Formel I gemäß Anspruch 1, in der

$R_1$ eine gegebenenfalls durch ein Fluor-oder Chloratom, durch eine Methyl-oder Methoxygruppe substituierte Phenylgruppe und

$R_2$ eine Hydroxycarbonylmethyl-, 3-Hydroxycarbonyl-n-propan-1-on-(1)-yl-oder 4,5-Dihydro-pyridazin-3-on-6-yl-gruppe und

X eine Methyliminogruppe, ein Sauerstoff-oder Schwefelatom bedeuten, deren Enantiomere und deren Salze mit anorganischen oder organischen Basen, falls $R_2$ eine Hydroxycarbonylgruppe enthält.

4. 4-[2-(2-(p-Chlorbenzolsulfonylamino)-äthyl)-1-methylpyrrol-5-yl]-4-oxobuttersäure und dessen Additionssalze.

5. 4-[2-(2-(p-Fluorbenzolsulfonylamino)-äthyl)-1-methyl-pyrrol-5-yl]-4-oxobuttersäure und dessen Additionssalze.

6. Physiologisch verträgliche Additionssalze der Verbindung gemäß den Ansprüchen 1 bis 5 mit anorganischen oder organischen Basen.

7. Arzneimittel, enthaltend als Wirkstoff eine Verbindung gemäß den Ansprüchen 1 bis 5 oder dessen physiologisch verträgliches Additionssalz gemäß Anspruch 6 neben gegebenenfalls einem oder mehreren inerten Trägerstoffen und/oder Verdünnungsmitteln.

8. Arzneimittel gemäß Anspruch 7 geeignet zur Behandlung und zur Prophylaxe thrombo-embolischer Erkrankungen, zur Prophylaxe der Arteriosklerose und zur Metastasenprophylaxe.

9. Verfahren zur Herstellung eines Arzneimittels gemäß den Ansprüchen 7 und 8, dadurch gekennzeichnet, daß auf nichtchemischem Wege eine Verbindung gemäß den Ansprüchen 1 bis 5 oder dessen physiologisch verträgliches Additionssalz gemäß Anspruch 6 in einen oder mehrere inerte übliche Trägerstoffe und/oder Verdünnungsmittel eingearbeitet wird.

10. Verfahren zur Herstellung der Verbindungen gemäß den Ansprüchen 1 bis 6, dadurch gekennzeichnet, daß

a.) eine Verbindung der allgemeinen Formel

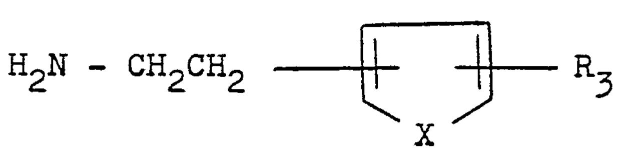

(II)

in der

$R_3$ die für $R_2$ in den Ansprüchen 1 bis 5 erwähnten Bedeutungen aufweist, wobei jedoch eine Hydroxygruppe im Rest $R_2$ durch eine hydrolytisch oder hydrogenolytisch abspaltbare Schutzgruppe geschützt sein kann, mit einem Phenylsulfonsäurederivat der allgemeinen Formel

$R_1-SO_2Y_1$ ,(III)

in der
$R_1$ wie in den Ansprüchen 1 bis 5 definiert ist und
$Y_1$ eine nucleophile Austrittsgruppe darstellt, umgesetzt und gegebenenfalls anschließend ein verwendeter Schutzrest abgespalten wird oder

b.) zur Herstellung von Verbindungen der allgemeinen Formel I, in der $R_2$ eine Hydroxycarbonylgruppe enthält, ein Schutzrest von einer Verbindung der allgemeinen Formel

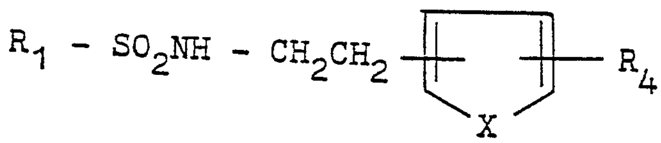

(IV)

in der
$R_1$ wie in den Ansprüchen 1 bis 5 definiert ist und $R_4$ die für $R_2$ in den Ansprüchen 1 bis 5 erwähnten Bedeutungen besitzt, wobei jedoch die Carboxygruppe durch einen hydrolytisch, thermolytisch oder hydrogenolytisch abspaltbare Schutzgruppe geschützt ist oder ein funktionelles Derivat der Carboxygruppe darstellt, abgespalten wird oder

c.) zur Herstellung von Verbindungen der allgemeinen Formel I, in der $R_2$ benachbart zum heterocyclischen Rest eine Carbonylgruppe enthält, eine Verbindung der allgemeinen Formel

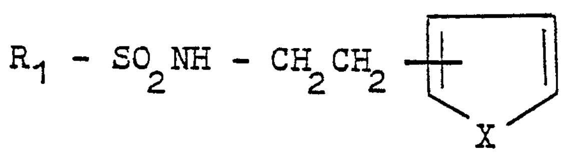

(V)

in der
$R_1$ wie in den Ansprüchen 1 bis 5 definiert ist, mit einer Verbindung der allgemeinen Formel

$Y_2-R_5$ ,(VI)

in der
$R_5$ die für $R_2$ in den Ansprüchen 1 bis 5 erwähnten Bedeutungen besitzt, wobei jedoch $R_2$ benachbart zu $Y_2$ eine Carbonylgruppe enthalten muß und gleichzeitig eine gegebenenfalls vorhandene Hydroxycarbonylgruppe durch einen hydrolytisch oder hydrogenolytisch abspaltbaren Schutzrest geschützt sein kann, und

$Y_2$ eine nucleophile Austrittsgruppe darstellen, oder dessen Anhydrid in Gegenwart einer Lewis-Säure acyliert und gegebenenfalls anschließend ein verwendeter Schutzrest abgespalten wird oder

d.) zur Herstellung von Verbindungen der allgemeinen Formel I, in der $R_2$ einen Pyridazinonring darstellt, eine Verbindung der allgemeinen Formel

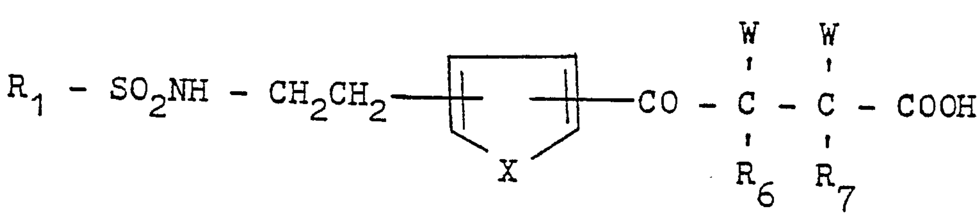

(VII)

in der
$R_1$ wie in den Ansprüchen 1 bis 5 definiert ist,
einer der Reste $R_6$ oder $R_7$ ein Wasserstoffatom oder eine Alkylgruppe mit 1 bis 3 Kohlenstoffatomen und der andere der Reste $R_6$ oder $R_7$ ein Wasserstoffatom und W jeweils ein Wasserstoffatom oder zusammen eine weitere Bindung darstellen, oder deren reaktionsfähige Derivate wie deren Ester, Amide oder Halogenide mit Hydrazin umgesetzt wird oder

e) zur Herstellung von Verbindungen der allgemeinen Formel I, in der $R_2$ eine über eine Methylengruppe gebundene Hydroxycarbonyl-oder Alkoxycarbonylgruppe darstellt, eine Verbindung der allgemeinen Formel

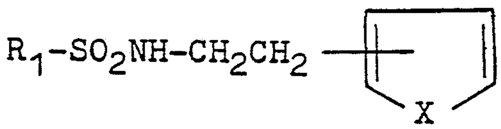 ,(VIII)

in der
$R_1$ und X wie in den Ansprüchen 1 bis 5 definiert sind, mit einem Diazoessigester der allgemeinen Formel

$N_2CH\text{-}COOR_8$ ,(IX)

in der
$R_8$ eine Alkylgruppe mit 1 bis 3 Kohlenstoffatomen darstellt, umgesetzt und eine so erhaltene Verbindung gegebenenfalls anschließend hydrolysiert wird und

gewünschtenfalls anschließend eine so erhaltene Verbindung der allgemeinen Formel I, in der $R_2$ eine Hydroxycarbonylgruppe enthält, mittels Veresterung in eine entsprechende Alkoxycarbonylverbindung übergeführt wird und/oder

eine so erhaltene Verbindung der allgemeinen Formel I, in der $R_2$ ein optisch aktives Kohlenstoffatom enthält, in ihre Enantiomeren aufgetrennt wird und/oder
eine so erhaltene Verbindung der allgemeinen Formel I, welche eine Carboxygruppe enthält, in ihre Additionssalze mit anorganischen oder organischen Basen, insbesondere für die pharmazeutische Anwendung in ihre physiologisch verträglichen Additionssalze, übergeführt wird.

11. Neue Verbindungen der allgemeinen Formel

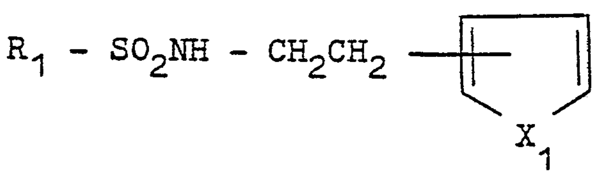 ,(X)

in der
$R_1$ wie in den Ansprüchen 1 bis 5 definiert ist und
$X_1$ eine gegebenenfalls durch eine Alkylgruppe mit 1 bis 3 Kohlenstoffatomen substituierte Iminogruppe oder ein Sauerstoffatom darstellt.

12. Verfahren zur Herstellung der neuen Verbindungen gemäß Anspruch 11, dadurch gekennzeichnet, daß eine Verbindung der allgemeinen Formel

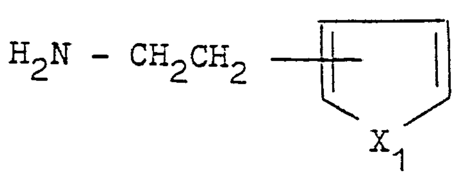 ,(XI)

in der
$X_1$ wie im Anspruch 11 definiert ist, mit einem Phenylsulfonsäurederivat der allgemeinen Formel

$R_1\text{-}SO_2Y_1$ ,(III)

in der
$R_1$ wie in den Ansprüchen 1 bis 5 definiert ist und $Y_1$ eine nucleophile Austrittsgruppe darstellt.